# FASCICULE DE BREVET EUROPEEN

(11) **EP 4 167 898 B1**
(45) Date de publication et mention de la délivrance du brevet: **24.04.2024**
(21) Numéro de dépôt: 21735685.6
(22) Date de dépôt: 23.06.2021
(51) Int. Cl.: A61C 7/00

(54) **PROCÉDÉ DE FABRICATION D'UN APPAREIL ORTHODONTIQUE**
VERFAHREN ZUR HERSTELLUNG EINER ORTHODONTISCHEN VORRICHTUNG
METHOD FOR MANUFACTURING AN ORTHODONTIC APPLIANCE

(30) Priorité: 23.06.2020 FR 2006550
(43) Date de publication de la demande: 26.04.2023
(73) Titulaire: Bergeyron, Patrice, 1201 Geneve (CH)
(72) Inventeur: Bergeyron, Patrice, 1201 Geneve (CH)
(74) Mandataire: Cabinet Nony
(86) Numéro de dépôt international: PCT/EP2021/067259
(87) Numéro de publication internationale: WO 2021/260076

(56) Documents cités:
- EP-A1- 3 566 673
- WO-A1-2019/040927
- US-A1- 2004 197 727
- US-A1- 2015 265 374

## Description

### Domaine technique

La présente invention concerne un procédé de fabrication d'un appareil orthodontique.

### Technique antérieure

Un traitement orthodontique met en oeuvre un appareil orthodontique pour déplacer une ou plusieurs dents d'un patient. Cependant, la conception de l'appareil orthodontique est un travail très complexe et même lorsque l'orthodontiste est expérimenté, il lui est toujours impossible de prévoir l'effet exact d'un appareil orthodontique. Il est ainsi fréquent que le patient ne soit pas satisfait du résultat obtenu, de la durée, de la douleur, ou du coût du traitement orthodontique ou encore du nombre de rendez-vous chez l'orthodontiste que le traitement orthodontique a impliqué.

US2004/197727 divulgue un procédé de fabrication d'au moins un appareil orthodontique destiné à être porté par un patient.

Il existe donc un besoin pour un procédé de fabrication d'un appareil orthodontique conduisant à un résultat plus conforme aux souhaits du patient.

Par ailleurs, la conception de l'appareil orthodontique est un travail long et il existe un besoin pour un procédé permettant de fabriquer plus rapidement un appareil orthodontique.

Un but de l'invention est de répondre, au moins partiellement, à ces besoins.

### Exposé de l'invention

### Résumé de l'invention

L'invention propose un procédé de fabrication d'au moins un appareil orthodontique destiné à être porté par un patient, pour la mise en oeuvre, depuis un instant initial jusqu' à un instant final, d'un traitement orthodontique complexe constitué d'au moins un premier traitement orthodontique élémentaire configuré pour modifier la morphologie de la tête du patient, de préférence de plusieurs dits premiers traitements orthodontiques élémentaires, et d'au moins un deuxième traitement orthodontique élémentaire configuré pour modifier la configuration des dents du patient, ledit procédé comportant les étapes comme définis dans la revendication 1.

De préférence, tout premier traitement orthodontique élémentaire est paramétré avant tout deuxième traitement orthodontique élémentaire. Autrement dit, tous les premiers traitements orthodontiques élémentaires pour modifier la morphologie de la tête du patient sont paramétrés avant les deuxièmes traitements orthodontiques élémentaires pour modifier la configuration des dents du patient. La priorité est donc donnée à la morphologie de la tête, et les deuxièmes traitements orthodontiques élémentaires ne peuvent donc, avantageusement, conduire à une morphologie de la tête inacceptable.

Comme on le verra plus en détail dans la suite de la description, la fabrication d'un appareil orthodontique selon l'invention n'est plus exclusivement guidée par l'objectif de modifier la configuration des dents, par exemple dans le seul but de résoudre une malocclusion. Elle doit aussi, et même prioritairement, respecter des règles relatives à la morphologie de la tête. Le risque que le traitement orthodontique complexe déforme la morphologie de la tête de manière inappropriée, et donc que le patient ne soit pas satisfait du résultat obtenu, est donc réduit, voire supprimé. En outre, le premier traitement orthodontique élémentaire peut avantageusement modifier la morphologie de la tête du patient d'une manière qui réponde aux besoins du patient, c'est-à-dire apporter une satisfaction supplémentaire au patient.

L'invention propose une démarche structurée, assistée par ordinateur, prenant systématiquement en compte l'effet de différents traitements orthodontiques élémentaires pour s'assurer que leur paramétrage ne remet pas en cause la conformité du traitement orthodontique complexe au règlement. Cette démarche conduit à un traitement orthodontique et à un appareil orthodontique mieux adaptés aux besoins du patient. Le nombre de rendez-vous chez l'orthodontiste en est avantageusement limité.

Le procédé selon l'invention peut comporter une ou plusieurs des caractéristiques optionnelles suivantes :
- l'instant final est postérieur à l'instant initial de plus de 2 semaines, 1 mois, trois mois et/ou de moins de 5 ans, de moins de 3 ans, ou de moins d'un an ;
- le traitement orthodontique complexe comporte de préférence plus de 2, plus de 5, plus de 10, et/ou moins de 100 traitements orthodontiques élémentaires, de préférence choisi(s) dans le groupe constitué par
   - un traitement de déplacement de la mâchoire inférieure vers le haut ou vers le bas ;
   - un traitement de modification de la largeur de la mâchoire supérieure ;
   - un traitement de modification de la largeur de la mâchoire inférieure ;
   - un traitement de déplacement de la mâchoire inférieure vers l'avant ou vers l'arrière;
   - un traitement de déplacement de la mâchoire supérieure vers l'avant ou vers l'arrière ;
   - un traitement de déplacement latéral de la mâchoire inférieure ;
   - un traitement de modification de l'inclinaison transversales des mâchoires ;
   - un traitement de déplacement d'une ou plusieurs dents ;
   - traitement de déplacement des gencives ;
   - un traitement de modification de la couleur d'une ou plusieurs dents, de préférence de blanchissement des dents.
- le nombre et/ou la nature des traitements orthodontiques élémentaires qui constituent le traitement orthodontique complexe est modifiable par un utilisateur, de préférence par l'orthodontiste ;
- pour constituer le traitement orthodontique complexe non paramétré, c'est-à-dire avant l'étape b) de paramétrage, l'utilisateur choisit des traitements orthodontiques élémentaires dans une base de modèles de traitements orthodontiques élémentaires non paramétrés, et les ordonne ;
- la base comporte un ou plusieurs modèles pour un ou plusieurs traitements orthodontiques élémentaires choisis dans ledit groupe ;
- les traitements orthodontiques élémentaires sont ordonnés pour être paramétrés suivant l'ordre de paramétrage suivant :
   - paramétrage d'un traitement de déplacement de la mâchoire inférieure, de préférence comportant un paramétrage d'un traitement de déplacement vertical de la mâchoire inférieure ;
   - paramétrage d'un traitement de modification de la largeur de la mâchoire supérieure ;
   - paramétrage d'un traitement de modification de la largeur de la mâchoire inférieure ;
   - paramétrage d'un traitement de déplacement avant/arrière de la mâchoire inférieure ;
   - paramétrage d'un traitement de déplacement avant/arrière de la mâchoire supérieure ;
   - paramétrage d'un traitement de déplacement droite/gauche de la mâchoire inférieure ;
   - paramétrage d'un traitement d'inclinaison des mâchoires ; paramétrage d'un traitement de déplacement des gencives ;
   - paramétrage d'un traitement de déplacement d'une ou plusieurs dents ;
   un ou plusieurs de ces traitements orthodontiques élémentaires pouvant être absents.
- l'ordre de paramétrage est défini, et de préférence modifiable par un utilisateur, de préférence par le patient et/ou l'orthodontiste, de préférence par l'orthodontiste ;
- le règlement définit :
   - au moins une règle capitale, c'est-à-dire relative à la morphologie de la tête, portant sur une valeur choisie dans le groupe constitué par
      - une hauteur de visage à l'instant final,
      - une hauteur de la mâchoire inférieure et/ou une hauteur de la mâchoire supérieure à l'instant final
      - une largeur de la mâchoire inférieure à l'instant final,
      - une position de la mâchoire inférieure par rapport à la mâchoire supérieure, à l'instant final,
      - une largeur de la mâchoire supérieure à l'instant final,
      - une position de la lèvre inférieure et/ou de la lèvre supérieure par rapport au menton et au front ;
   - au moins une règle dentaire, c'est-à-dire relative à la configuration des dents, de préférence portant sur une valeur choisie dans le groupe constitué par
      - une distance par rapport à une configuration cible des dents à l'instant final ;
      - une inclinaison par rapport à une configuration cible des dents à l'instant final ;
      - un déplacement des gencives par rapport à une configuration cible des dents à l'instant final ;
      - une couleur cible des dents à l'instant final ;
   - au moins une règle transversale, c'est-à-dire qui n'est ni une règle capitale, ni une règle dentaire, de préférence portant sur une valeur choisie dans le groupe constitué par
      - un coût pour le traitement orthodontique complexe,
      - une durée pour le traitement orthodontique complexe,
      - un coefficient de douleur pour le traitement orthodontique complexe,
      - un coefficient de confort pour le traitement orthodontique complexe,
      - une ou plusieurs caractéristiques techniques pour ledit au moins un appareil orthodontique ;
- on détermine, partiellement ou complètement, le règlement, de préférence au moins une partie des règles transversales, en fonction du patient, de préférence en fonction de choix du patient ;
- on détermine, partiellement ou complètement, le règlement, de préférence au moins une partie des règles capitales, à partir d'images, de préférence des photos, de préférence en couleurs et de préférence en couleurs réalistes et/ou à partir de modèles 3D, de préférence un modèle 3D de la face, et/ou un modèle 3D de l'intérieur de la bouche, dit « intraoral », en particulier modèle 3D de dentition et/ou un cliché tomographique ou « cone beam » (CBCT);
- lesdites images comprennent de préférence au moins
   - une image de face et une image de profil de la tête, et en particulier du visage du patient, et/ou
   - une image représentant la bouche du patient en position ouverte et au moins une image représentant la bouche du patient en position fermée, et/ou
   - une image représentant la bouche du patient en position de sourire, ou « image de sourire », de préférence deux images représentant la bouche du patient en position de sourire comportant une images de face et une profil, et/ou
   - un cliché panoramique des arcades dentaires, et/ou
   - une radiographie de la tête, de préférence au moins une radiographie de profil de la tête du patient;
- lesdits modèles 3D comprennent de préférence au moins un scanner facial, c'est-à-dire un modèle 3D de la face du patient ;
- une règle définit, pour une dimension de la tête ou des dents, une plage de valeurs acceptables pour ladite dimension de la tête ou des dents à l'instant final ;
- de préférence au moins une partie des règles capitales et/ou dentaires définissent chacune une plage de valeurs acceptables, dont les bornes sont définies au moyen d'un référentiel, pour une dimension de la tête ou des dents à l'instant final ;
- lesdites dimensions sont déterminées à partir desdites images et/ou desdits modèles 3D, de préférence par une distance entre des points remarquables desdites images et/ou desdits modèles 3D ;
- l'utilisateur, de préférence l'orthodontiste, identifie lesdits points remarquables sur les images et/ou les modèles 3D affichés sur l'écran de l'ordinateur, dispose des marques de mesure en fonction des points remarquables, et l'ordinateur détermine lesdites dimensions en fonction de la disposition desdites marques de mesure ;
- alternativement, les images et/ou les modèles 3D sont analysés informatiquement, de préférence au moyen d'une méthode d'apprentissage automatique, de préférence au moyen d'un réseau de neurones, pour déterminer lesdites marques de mesure et/ou lesdits points remarquables et/ou lesdites dimensions ;
- des points remarquables sont de préférence choisis dans le groupe constitué par
   o le centre d'un oeil du patient,
   o un point inter-arcades sourcilières, de préférence situé à mi-distance entre les sourcils,
   ∘ un point sous-nasal, en particulier situé au centre sous le nez,
   ∘ un point frontal, en particulier situé sur la ligne d'implantation des cheveux, en particulier au milieu du front, et/ou le point le plus proéminent du front sur une vue de profil,
   ∘ le point à l'extrémité la plus à droite du visage du patient vu de face,
   ∘ le point à l'extrémité la plus à gauche du visage du patient vu de face,
   ∘ un point situé sur l'extrémité du menton, de préférence au milieu du menton, et/ou le point le plus proéminent du menton sur une vue de profil,
   ∘ un point situé sur une commissure des lèvres du patient,
   ∘ un point situé sur le contour des lèvres, en particulier sur l'extrémité de la lèvre supérieure et/ou l'extrémité de la lèvre inférieure, de préférence le point le plus proéminent,
   ∘ un point situé sur la jonction des lèvres lorsqu'elles sont fermées, en particulier, le centre de la bouche lorsque les lèvres sont fermées,
   ∘ un point situé à la jonction entre deux dents, en particulier entre les incisives centrales,
   ∘ un point situé sur le contour d'une dent, en particulier un point situé au milieu du contour inférieur d'une dent supérieure et/ou un point situé au milieu du contour supérieur d'une dent inférieure, par exemple une pointe cuspidienne d'une dent ou un creux entre deux cuspides,
   ∘ un point de contact entre deux dents, en particulier entre une dent supérieure et une dent inférieure,
   ∘ un point de jonction entre l'émail et la gencive d'une dent,
   ∘ le porion cutané,
   ∘ l'orbital cutané.
- des points remarquables définissent ou sont utilisés pour définir ledit référentiel ;
- l'utilisateur, de préférence l'orthodontiste, identifie lesdits points remarquables sur les images et/ou les modèles 3D affichés sur l'écran de l'ordinateur, dispose des marques de référence en fonction des points remarquables, et l'ordinateur détermine le référentiel en fonction de la disposition desdites marques de référence ;
- les marques de référence sont de préférence choisies dans le groupe constitué par un point, une droite, un segment, et un cercle ;
- alternativement, les images et/ou les modèles 3D sont analysés informatiquement, de préférence au moyen d'une méthode d'apprentissage automatique, de préférence au moyen d'un réseau de neurones, pour déterminer le référentiel ;
- le règlement peut être modifié à un instant lors du paramétrage, de préférence à tout instant lors du paramétrage (étape b)), dans la mesure où le traitement orthodontique complexe, tel que paramétré jusqu'à cet instant, reste conforme au règlement ;
- de préférence, pour paramétrer un traitement orthodontique élémentaire, on choisit, de préférence l'orthodontiste choisit un bloc de paramétrage dans un groupe de blocs de paramétrage potentiels potentiellement applicables au traitement orthodontique élémentaire pour le paramétrer, de préférence en sélectionnant un desdits blocs de paramétrage potentiels dans une liste présentée sur l'écran de l'ordinateur, l'ordinateur paramétrant ensuite le traitement orthodontique élémentaire avec ledit bloc de paramétrage ;
- ledit groupe de blocs de paramétrage potentiels présenté à l'utilisateur, de préférence l'orthodontiste, ne comporte que des blocs de paramétrage compatibles avec le règlement, c'est-à-dire qui, appliqués au traitement orthodontique élémentaire, conduisent à un traitement orthodontique complexe respectant le règlement ;
- à un instant lors du paramétrage, de préférence à tout instant lors du paramétrage, on présente, de préférence sur l'écran de l'ordinateur, et de préférence on met à jour en temps réel, des informations relatives
   - au règlement, par exemple le coût maximal pour le traitement orthodontique complexe, et/ou
   - à des paramètres du traitement orthodontique complexe, par exemple le coût du traitement orthodontique complexe tel que paramétré audit instant, et/ou
   - à la morphologie finale, c'est-à-dire à l'instant final, auquel le traitement orthodontique complexe tel que paramétré audit instant conduirait (c'est-à-dire à la morphologie que l'on anticipe à l'instant final si le traitement orthodontique complexe était mis en oeuvre), et/ou
   - à la configuration finale des dents, c'est-à-dire à l'instant final, auquel le traitement orthodontique complexe tel que paramétré audit instant conduirait ;
- lesdites informations comportent une représentation, de préférence réaliste, des dents et/ou de la tête du patient dans la configuration finale des dents et/ou la morphologie finale, respectivement ;
- pour vérifier, à un instant de l'étape b), si le traitement orthodontique complexe respecte le règlement, on mesure, de préférence à l'étape a), des dimensions sur des images et/ou des modèles 3D affichés sur un écran d'ordinateur, entre des points remarquables desdites images et/ou desdits modèles 3D, lesdites dimensions constituant des valeurs initiales de paramètres du traitement orthodontique complexe, on simule l'effet du traitement orthodontique complexe, tel que paramétré audit instant de l'étape b), sur lesdites dimensions, entre l'instant initial et l'instant final, c'est-à-dire en anticipant l'effet du traitement orthodontique complexe sur ces dimensions entre l'instant initial et l'instant final, de manière à déterminer des valeurs finales pour lesdits paramètres, puis on vérifie si lesdites valeurs finales appartiennent à des plages correspondantes définies par les règles du règlement ;
- pour déterminer lesdites valeurs initiales,
   - on identifie des points remarquables sur les images et/ou les modèles 3D, et on dispose des marques de mesure en fonction des points remarquables, ledit ordinateur déterminant lesdites valeurs initiales en fonction de la disposition desdites marques de mesure, ou
   - les images et/ ou les modèles 3D sont analysés informatiquement au moyen d'une méthode d'apprentissage automatique pour déterminer lesdites valeurs initiales ;
- pour définir au moins une borne d'une dite plage relative à une position d'un point remarquable du patient, on dispose sur au moins une dite image et/ou un dit modèle 3D au moins une marque de référence, la position de la marque de référence indiquant une position extrême pour ledit point remarquable à l'instant final, et définissant ainsi ladite borne ;
- ledit au moins un appareil orthodontique est une gouttière orthodontique ;
- après l'étape c), on génère un compte rendu comportant des informations relatives
   - au règlement, et/ou
   - à des paramètres du traitement orthodontique complexe, et/ou
   - à une morphologie auquel le traitement orthodontique complexe conduira à l'instant final, et/ou
   - à une configuration finale des dents auquel le traitement orthodontique complexe conduira à l'instant final ;
- ledit compte-rendu est présenté à l'orthodontiste et/ou au patient, par exemple en étant envoyé ou en étant affiché, par exemple sur un écran ;
- ledit paramétrage est présenté à un utilisateur pour former ledit utilisateur à l'orthodontie.

L'invention concerne aussi
- un programme informatique comprenant des instructions de code pour l'exécution, au moins partielle, d'une ou plusieurs étapes a), b), et c), de préférence de toutes ces étapes lorsque ledit programme est exécuté par un ordinateur,
- un support informatique sur lequel est enregistré un tel programme, par exemple une mémoire ou CD-ROM, et
- un ordinateur sur lequel est chargé un tel programme.

L'invention concerne aussi un kit comportant :
- un appareil d'acquisition des images et/ou des modèles 3D, comportant par exemple une caméra, un appareil photo, et/ou un scanner de préférence 3D et/ou un « cône beam »;
- un ordinateur chargé avec un programme informatique selon l'invention.

### Définitions

Un « patient » est une personne pour laquelle un procédé selon l'invention est mis en oeuvre, indépendamment du fait que cette personne soit malade ou non.

Par « orthodontiste », on entend toute personne qualifiée pour prodiguer des soins orthodontiques, ce qui inclut également un dentiste.

L'utilisateur est la personne qui met en oeuvre le procédé, classiquement l'orthodontiste, de préférence avec le patient.

Un « écarteur » (« retractor » en anglais), ou « écarteur dentaire », est un dispositif destiné à retousser les lèvres. Il comporte un rebord supérieur et un rebord inférieur s'étendant autour d'une ouverture d'écarteur. En position de service, les lèvres supérieure et inférieure du patient sont en appui sur les rebords supérieur et inférieur, respectivement. L'écarteur est configuré de manière à écarter élastiquement l'une de l'autre les lèvres supérieure et inférieure de manière à dégager les dents visibles à travers l'ouverture. Un écarteur permet ainsi d'observer les dents sans être gêné par les lèvres.

Par « image » on entend une image en deux dimensions comme une photographie ou une image extraite d'un film. Une image est formée de pixels. Une « photo » est une image qui représente un objet réel comme le perçoit l'oeil humain, de préférence en couleurs réalistes. Un cliché tomographique ou un panoramique acquis par rayons X sont des images qui ne sont pas des photos. Une image peut également être une vue d'un modèle 3D. Un modèle 3D, est un modèle tridimensionnel numérique. Il est constitué d'un ensemble de voxels. Par « appareil d'acquisition », on entend tout appareil permettant de prendre une image, ce qui inclut une caméra, un téléphone mobile, une tablette ou un ordinateur.

Par « image capitale », on entend une image représentant au moins une partie de la tête du patient.

Par « image dentaire », on entend une image représentant au moins une partie des dents du patient.

Une « règle » définit une contrainte pour le traitement orthodontique complexe, par exemple « le coût doit être inférieur à 10 000 € », « la mâchoire inférieure doit être avancée d'une distance comprise entre 1 et 5 mm », « la dent n°17 doit être tournée, autour de son axe, de 15° ». Les règles définissent ainsi les plages de valeurs qu'un paramètre du traitement orthodontique complexe peut prendre.

Une configuration des dents ou une morphologie de la tête « cible » des dents sont un agencement des dents ou une forme de la tête que l'on souhaite atteindre à l'instant final. Le règlement peut définir que la configuration des dents ou la morphologie de la tête cible doit être atteinte à l'instant final, éventuellement avec une marge de tolérance.

Un « traitement orthodontique élémentaire » est un traitement orthodontique destiné à agir, par l'utilisation d'un appareil orthodontique, sur la morphologie de la tête et/ou la configuration des dents. Il est défini par cette action, ou « fonction ». Il devient opérationnel après paramétrage, c'est-à-dire après application d'un bloc de paramétrage.

Classiquement, un traitement orthodontique élémentaire est destiné à rapprocher au moins une valeur d'un paramètre d'une valeur cible correspondante, de préférence de manière que ladite valeur atteigne la valeur cible à la fin du traitement orthodontique élémentaire.

On distingue en particulier les traitements orthodontiques élémentaires « capitaux », qui agissent essentiellement sur la morphologie de la tête, et les traitements orthodontiques élémentaires « dentaires », qui agissent essentiellement sur la configuration des dents. Un traitement orthodontique élémentaire dentaire peut agir sur la morphologie de la tête et, réciproquement, un traitement orthodontique élémentaire capital peut agir sur la configuration des dents. Cependant, tout traitement orthodontique élémentaire a une action prépondérante, sur la morphologie de la tête ou sur la configuration des dents, ce qui permet de le classer parmi les traitements orthodontiques élémentaires capitaux ou dentaires.

Les traitements orthodontiques élémentaires capitaux comprennent en particulier
- un traitement de modification de la position de la mâchoire inférieure par rapport à la mâchoire supérieure,
- un traitement de déplacement de la mâchoire inférieure vers le haut ou le bas ;
- un traitement de modification de la largeur de la mâchoire supérieure ;
- un traitement de modification de la largeur de la mâchoire inférieure ;
- un traitement de déplacement de la mâchoire inférieure vers l'avant ou vers l'arrière;
- un traitement de déplacement de la mâchoire supérieure vers l'avant ou vers l'arrière;
- un traitement de déplacement latéral de la mâchoire inférieure ;
- un traitement de modification de la largeur de la mâchoire supérieure,
- un traitement de modification de la largeur de la mâchoire inférieure,
- un traitement d'inclinaison des mâchoires.

Un traitement orthodontique élémentaire capital n'implique pas nécessairement une modification de la morphologie de la tête du patient. Par exemple, si le règlement impose une position de la mâchoire inférieure qui est celle à l'instant initial, c'est-à-dire qu'il définit une plage de valeurs pour le paramètre « position de la mâchoire inférieure » qui est limitée à la valeur initiale pour ce paramètre, le traitement orthodontique élémentaire capital doit être paramétré pour assurer que la position de la mâchoire inférieure ne sera pas modifiée par le traitement orthodontique complexe. Au moins le premier traitement orthodontique élémentaire, capital, implique une modification de la morphologie de la tête du patient.

Les traitements orthodontiques élémentaires dentaires comprennent en particulier
- un traitement de modification de la configuration des dents, en particulier un traitement de déplacement et/ou d'inclinaison d'au moins une dent,
- un traitement de déplacement des gencives,
- un traitement de modification de la couleur des dents.

On distingue les traitements orthodontiques élémentaires, bien connus, du traitement orthodontique complexe qui est un assemblage de traitements orthodontiques élémentaires. Un traitement orthodontique complexe peut comporter un ou plusieurs premiers traitements orthodontiques élémentaires et un ou plusieurs deuxièmes traitements orthodontiques élémentaires.

Pour réaliser un traitement orthodontique élémentaire, une ou plusieurs solutions sont possibles. Chacune de ces solutions est considérée comme le résultat d'un paramétrage particulier du traitement orthodontique élémentaire. Un bloc de paramétrage comporte l'ensemble des informations qui permettent de paramétrer le traitement orthodontique élémentaire pour constituer une solution particulière, c'est-à-dire un mode de réalisation particulier, ou « version », du traitement orthodontique élémentaire. Le paramétrage consiste à fixer une valeur ou une plage de valeurs acceptables pour au moins un paramètre d'un traitement orthodontique élémentaire, c'est-à-dire à établir des règles spécifiques à un traitement orthodontique élémentaire, à la différence des règles du règlement, qui concernent le traitement orthodontique complexe. Dans un mode de réalisation, le paramétrage d'un traitement orthodontique élémentaire consiste à sélectionner un bloc de paramétrage dans un groupe de blocs de paramétrage dits « potentiels ». Le bloc de paramétrage sélectionné est dit « actif ».

Les règles du règlement concernent le traitement orthodontique complexe, mais plusieurs traitements orthodontiques élémentaires peuvent contribuer à modifier la valeur d'un paramètre associé à une règle. Par exemple, une règle peut imposer un avancement de la mâchoire inférieure de 3 mm et plusieurs traitements orthodontiques élémentaires peuvent avoir un effet sur la position de la mâchoire inférieure. L'ordre de paramétrage conduit à ce que le paramétrage d'un traitement orthodontique élémentaire réduise les possibilités de paramétrage des traitements orthodontiques élémentaires paramétrés ultérieurement.

A un instant de l'étape de paramétrage où un ou plusieurs traitements orthodontiques élémentaires ont été paramétrés, le traitement orthodontique complexe « paramétré » est celui qui comporte ce ou ces traitements orthodontiques élémentaires paramétrés. Le paramétrage du traitement orthodontique complexe se complète donc à mesure que les traitements orthodontiques élémentaires sont paramétrés.

« Capital » est utilisé, à des fins de clarté, pour faire référence à des images représentant au moins une partie de la tête du patient, à des paramètres relatifs à la morphologie de la tête, à des valeurs de ces paramètres, à des règles pour ces paramètres, à des traitements orthodontiques élémentaires adaptés pour modifier ces valeurs et à des blocs de paramétrage pour ces traitements orthodontiques élémentaires.

« Dentaire » est utilisé, à des fins de clarté, pour faire référence à des images représentant au moins une partie des dents du patient, à des paramètres relatifs à la position et/ou l'orientation des dents, à des valeurs de ces paramètres, à des règles pour ces paramètres, à des traitements orthodontiques élémentaires adaptés pour modifier ces valeurs et à des blocs de paramétrage pour ces traitements orthodontiques élémentaires.

Une « plage de valeurs » est définie par des bornes inférieure et supérieure. Elle peut ne contenir qu'une valeur, la borne inférieure étant alors égale à la borne supérieure.

Un « groupe » peut comporter un ou plusieurs éléments.

Par « ordinateur », on entend tout appareil électronique, ce qui inclut un ensemble de plusieurs machines, ayant des capacités de traitement informatique. Classiquement, un ordinateur comporte en particulier un processeur, une mémoire, une interface homme-machine, comportant classiquement un écran, un module de communication par internet, par WIFI, par Bluetooth^{®} ou par le réseau téléphonique. Un logiciel configuré pour mettre en oeuvre le procédé de l'invention est chargé dans la mémoire de l'ordinateur. L'ordinateur peut être également connecté à une imprimante.

L'ordinateur peut être un serveur à distance de l'utilisateur, par exemple être le « cloud ».

« Actif » (ou « activé ») qualifie un bloc de paramétrage appliqué à un traitement orthodontique élémentaire. Par extension, le traitement orthodontique élémentaire peut être également qualifié d'actif.

Il faut interpréter "comprenant " ou "comportant " ou "présentant " de manière non restrictive, sauf indication contraire.

### Brève description des dessins

D'autres caractéristiques et avantages apparaîtront encore à la lecture de la description détaillée qui va suivre et à l'examen du dessin annexé dans lequel :
[Fig 1] la figure 1 représente, schématiquement, différentes étapes d'un mode de réalisation d'un procédé de fabrication d'un appareil orthodontique selon l'invention ;
[Fig 2] la figure 2 représente un exemple d'image comportant des marques de mesures, en particulier pour la détermination de règles capitales et le paramétrage de traitements orthodontiques élémentaires capitaux ;
[Fig 3] la figure 3 représente un exemple de détermination d'images comportant des marques de mesures, en particulier pour la détermination de règles dentaires et le paramétrage de traitements orthodontiques élémentaires dentaires ;
[Fig 4] la figure 4 illustre un exemple de combinaison d'une photo de profil et d'une radiographie ; et
[Fig 5] la figure 5 illustre un compte-rendu relatif à un traitement orthodontique complexe paramétré selon l'invention.

### Description détaillée

La description détaillée qui suit est celle de modes de réalisation préférés, mais n'est pas limitative.

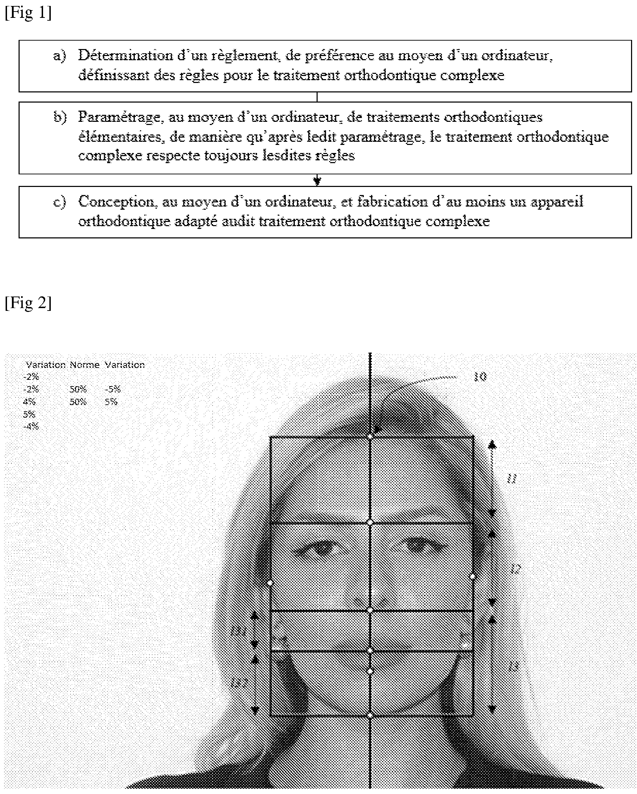

Un procédé de fabrication d'un appareil orthodontique comporte les étapes illustrées dans la figure 1. Il est assisté par ordinateur, de préférence mis en oeuvre par un ordinateur, à l'exception de l'acquisition des images et de la fabrication de l'appareil orthodontique.

Il repose sur le paramétrage structuré d'un traitement orthodontique complexe mettant en oeuvre un ou plusieurs appareils orthodontiques, entre des instants initial et final. En particulier, le traitement orthodontique complexe est conçu pour respecter prioritairement des règles capitales relatives à la forme, ou « morphologie » de la tête du patient. Pour paramétrer le traitement orthodontique complexe, les règles dentaires ne sont prises en compte qu'après qu'au moins une partie des règles capitales a été prise en compte.

**A l'étape a**), on définit le règlement, c'est-à-dire un ensemble de règles que doit respecter le traitement orthodontique complexe, quel que soit le niveau de son paramétrage, c'est-à-dire le nombre de traitements orthodontiques élémentaires ayant été paramétrés à l'étape b).

Le règlement peut être universel, ou être défini pour un groupe d'individus, par exemple pour un ensemble d'individus d'une même classe d'âge ou du même sexe ou partageant une même pathologie. Il est de préférence propre au patient.

Le règlement peut être défini par le patient et/ou l'orthodontiste.

Dans un mode de réalisation, le référentiel est initialement au moins en partie constitué de règles types, et la définition du règlement consiste à préciser ces règles types pour les rendre opérationnelles. Par exemple, une règle type peut être « le coût maximal pour le traitement orthodontique complexe est de X » et elle est rendue opérationnelle après que l'utilisateur a saisi la valeur de X, pour devenir par exemple la règle suivante : « le coût maximal pour le traitement orthodontique complexe est de 10 000 € ».

Autre exemple, une règle type est « la distance entre le point mentonnier et la droite reliant le point le plus proéminent de la lèvre inférieure au point le plus proéminent du front, sur une image représentant une vue de côté de la tête, doit être inférieure à Y » et elle est rendue opérationnelle après que l'utilisateur a saisi la valeur de Y, pour devenir la règle suivante : « la distance entre le point mentonnier et la droite reliant le point le plus proéminent de la lèvre inférieure au point le plus proéminent du front, sur une image représentant une vue de côté de la tête, doit être inférieure à 5 mm ».

Des règles peuvent être prédéfinies. Par exemple, une règle peut imposer, par défaut, « la distance entre le point mentonnier et la droite reliant le point le plus proéminent de la lèvre inférieure au point le plus proéminent du front, sur une image représentant une vue de côté de la tête, doit être inférieure à 10 mm ». De préférence, les règles prédéfinies peuvent être modifiées.

On peut distinguer, dans le règlement, les règles « capitales », « dentaires » et « transversales ».

### Règles transversales

Une « règle transversale » est une règle qui n'est ni une règle capitale, ni une règle dentaire. Les règles transversales sont de préférence déterminées par le patient.

De préférence, les règles transversales précisent un coût maximal et/ou une durée maximale et/ou une valeur maximale pour un coefficient de douleur et/ou une valeur minimale pour un coefficient de confort tenant de préférence compte du nombre de rendez-vous à prévoir chez l'orthodontiste, et/ou une caractéristique technique générale pour l'appareil orthodontique à fabriquer, par exemple en précisant que cet appareil orthodontique doit être une gouttière orthodontique.

Les règles transversales sont de préférence saisies dans l'ordinateur, par exemple avec un clavier.

### Règles capitales

Les « règles capitales » sont des règles qui imposent des plages, définies par le référentiel, pour les valeurs des paramètres capitaux, c'est-à-relatifs à la forme de la tête, à l'instant final. Dans un mode de réalisation, le référentiel définit une morphologie de la tête souhaitée par le patient et/ou l'orthodontiste, de préférence par le patient, c'est-à-dire une morphologie cible. La morphologie cible peut fixer une valeur pour au moins une partie des paramètres capitaux, voire pour chaque paramètre capital, ou autoriser une plage de plusieurs valeurs pour au moins une partie des paramètres capitaux, voire pour chaque paramètre capital.

La morphologie cible est différente de la morphologie de la tête à l'instant initial, c'est-à-dire que le règlement impose que le traitement orthodontique complexe modifie les dimensions de la tête du patient.

Un paramètre capital peut être en particulier une dimension ou un angle.

Un paramètre capital peut être en particulier choisi parmi :
- une distance entre deux points, par exemple un point situé au milieu du front au niveau de l'implantation capillaire, et un point inter-arcades sourcilières, ou entre un point inter-arcades sourcilières et un point sous nasal ou entre un point sous nasal et un point mentonnier ;
- une distance entre les deux commissures des lèvres du patient lorsqu'il sourit ;
- une largeur pour une dentition représentée sur une image de sourire ;
- une distance entre une droite passant par un point inter-arcades sourcilières et un point sous nasal, et le point inter-incisif des incisives centrales supérieures ;
- une distance entre une droite passant par la jonction des premières incisives supérieure et une droite passant par la jonction des premières incisives inférieures;
- un angle entre une droite passant par au moins deux points parmi un point inter-arcades sourcilières, un point sous nasal et un point inter-incisives et une droite passant par un point au niveau de la cuspide de la première prémolaire gauche, ou droite, et un point au niveau de la jonction de l'émail-cément de la première prémolaire gauche, ou droite respectivement ;

Le nombre de paramètres capitaux est de préférence supérieur à 2, 3, 5 et/ou inférieur à 50.

### Règles dentaires

Les « règles dentaires » sont des règles qui imposent des plages, définies par le référentiel, pour les valeurs des paramètres dentaires, c'est-à-relatifs à la configuration des dents, à l'instant final.

Les règles dentaires classiquement utilisées pour définir les traitements orthodontiques conventionnels peuvent être utilisés. Elles sont de préférence propres au patient, et de préférence déterminées par l'orthodontiste, en fonction des besoins du patient.

De préférence, une règle dentaire définit un écart, éventuellement nul, par rapport à la configuration des dents d'au moins une arcade orthodontique du patient telle que modélisée sur un modèle numérique tridimensionnel, dit « modèle cible ». Autrement dit, elle impose au moins une plage pour un paramètre de position d'une dent du patient.

Le modèle cible peut en particulier modéliser les dents du patient dans leur agencement souhaité à l'instant final, c'est-à-dire à la fin du traitement orthodontique complexe.

Le modèle cible peut être réalisé par modification d'un modèle numérique tridimensionnel de ladite arcade dentaire, de préférence réalisé moins de 3 mois avant l'instant initial, dit « modèle initial ». Le modèle initial est de préférence obtenu par un scan de ladite arcade.

De manière générale, les règles capitales et/ou les règles dentaires sont de préférence définies moins de 6 mois, moins de 3 mois, de préférence moins d'un mois, voire moins d'une semaine avant l'instant initial.

De préférence, le règlement est défini, au moins partiellement, à partir d'images acquises à un instant d'acquisition. L'instant d'acquisition est antérieur à l'étape a), de préférence de moins de 6 mois, 3 mois, 1 mois, de préférence de moins d'une semaine.

De préférence, au moins une partie du référentiel, en particulier les limites pour les variations des valeurs des paramètres capitaux et dentaires pendant le traitement orthodontique complexe, sont définies, au moins partiellement, avec des images.

### Images

De préférence, les images comportent des « images capitales », représentant au moins une partie de la tête du patient, et des « images dentaires », représentant au moins une partie des dents du patient. Lorsqu'une image capitale représente des dents, elle constitue également une image dentaire.

Les images, et en particulier les images capitales, contiennent de préférence des photos, de préférence en couleurs réalistes. Les photos peuvent être acquises à l'aide de tout appareil photographique.

De préférence, au moins une partie des images capitales, de préférence toutes les images capitales représentent plus de 50%, de préférence plus de 70%, plus de 80%, plus de 90%, de préférence 100% de la hauteur de la tête, depuis le menton jusqu'au sommet du crâne.

De préférence, les images comportent au moins une image de la tête du patient vue de face, une image de la tête vue de profil, et une image de sourire, de préférence une image de sourire vu de face. De préférence, les images comportent également au moins une image du patient avec la bouche ouverte et une image de patient avec la bouche fermée.

Les images peuvent comporter des photos, des radiographies, des vues d'un modèle tridimensionnel obtenu par un scan d'une arcade orthodontique, et/ou une combinaison de telles images.

Les images, et en particulier les images dentaires, contiennent de préférence des radiographies. Les radiographies sont de préférence réalisées par un professionnel de santé, par exemple un orthodontiste ou un laboratoire d'orthodontie.

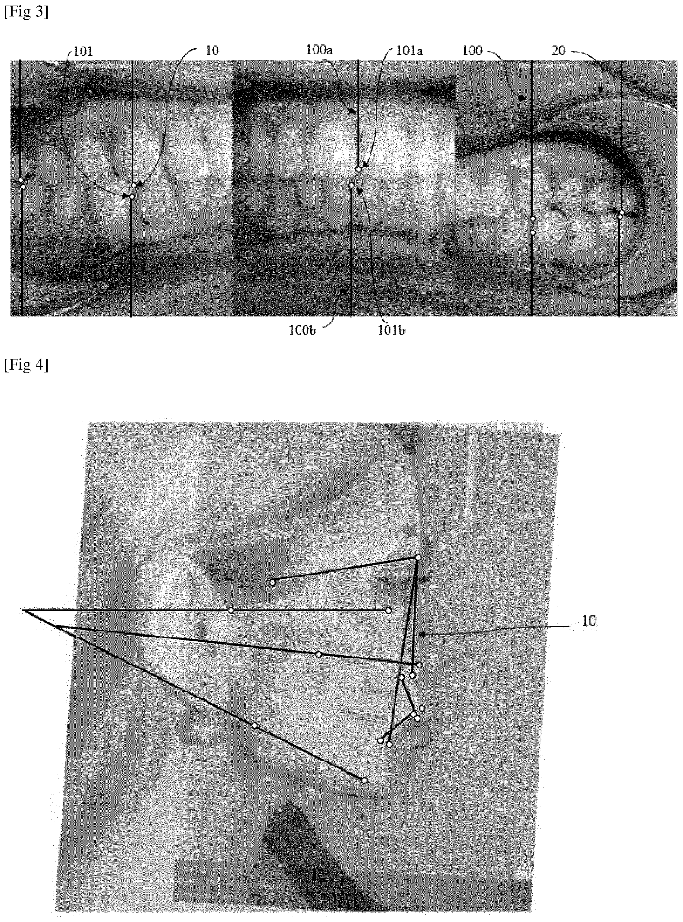

Les images contiennent de préférence au moins une image « combinée », c'est-à-dire résultant d'une combinaison de plusieurs images. De préférence, au moins une image combinée représente une superposition d'une photo, de préférence en couleurs réalistes, avec une radiographie, comme représenté sur l'exemple de la figure 4. La superposition est de préférence réalisée en augmentant la transparence d'au moins une des images à superposer, notamment en exploitant un programme informatique, les images à superposer représentant une même vue, par exemple une vue de face ou une vue de profil de la tête du patient.

De préférence, au moins une partie des images, et en particulier des images dentaires, sont des images extraorales qui représentent les dents du patient alors que le patient porte un écarteur 20, comme sur les images de la figure 3.

De préférence, des marques de référence sont placées sur les images, de préférence affichées sur l'écran de l'ordinateur, de préférence par l'utilisateur, de préférence un orthodontiste, pour définir des positions extrêmes identifiées par des points remarquables. Les images servent ainsi à saisir les limites pour les variations de ces positions.

La marque de référence peut être notamment une marque géométrique, de préférence choisie parmi un point, une droite, un cercle ou un arc de cercle.

De préférence, au moins une marque de référence est choisie dans le groupe constitué par
- au moins une paire de points, en particulier trois paires de points, par exemple une première paire comprenant un point situé au milieu du front au niveau de l'implantation capillaire et un point inter-arcades sourcilières, une deuxième paire comprenant un point inter-arcades sourcilières et un point sous nasal et une troisième paire comprenant un point sous nasal et un point mentonnier (une règle définissant le déplacement vertical de la mâchoire peut être déterminée à partir d'une ou plusieurs de ces trois paires de points) ;
- un point situés sur chacune des commissures des lèvres du patient lorsqu'il sourit sur une vue de face (une règle définissant la largueur d'arcade dentaire peut être déterminée à partir de ces deux points);
- deux points définis par les points les plus externes des dents les plus proches des commissures des lèvres, lorsque ces dents sont orientées parallèlement à l'axe vertical défini par un point inter-arcades sourcilières et un point sous nasal (une règle définissant une largeur de dentition maximale ou cible peut être définie à partir de ces deux points)
- une droite passant par un point inter-arcades sourcilières et un point sous nasal, et un point inter-incisif des incisives centrales supérieures (une règle définissant le déplacement latéral des incisives centrales peut être définie à partir de cette droite et de ce point) ;
- un point situé sur la jonction des premières incisives supérieures et un point situé sur la jonction des premières incisives inférieures (une règle définissant le déplacement relatif droite/gauche des incisives supérieures et inférieures peut être définie à partir de ces points) ;
- une droite passant par au moins deux points parmi un point inter-arcades sourcilières, un point sous nasal et/ou un point inter-incisives, et une droite passant par un point au niveau de la cuspide de la première prémolaire gauche, ou droite, et un point au niveau de la jonction de l'émail-cément de la première prémolaire gauche, ou droite respectivement (une règle définissant la rotation de la première prémolaire peut être définie) ;
- un arc de cercle passant par exemple par deux points situés sur les commissures des lèvres du patient vue de face et ayant pour centre un point situé au milieu de la lèvre inférieure ; une règle définissant le déplacement vertical d'au moins une des arcades dentaires peut être définie) ;
- une droite reliant la pointe du menton au point le plus proéminent du front, sur une image représentant une vue de côté de la tête (une règle délimitant le déplacement avant/arrière du menton peut être alors déterminée à partir de cette droite) ;

Les marques de référence peuvent être placées automatiquement grâce à une méthode d'analyse d'images, par exemple grâce à des méthodes d'apprentissage automatique, en particulier un réseau de neurones.

De préférence, des instructions sont données oralement ou affichées sur un écran afin que l'utilisateur positionne les marques de référence manuellement, par exemple à l'aide d'une souris, d'un stylet ou d'un doigt, par interaction avec un écran tactile de l'ordinateur.

De préférence, l'ordinateur impose un ordre pour placer les marques de référence, ce qui lui permet ensuite d'établir les règles en conséquence.

**A l'étape b**), les blocs de paramétrage des différents traitements orthodontiques élémentaires du traitement orthodontique complexe sont progressivement déterminés et activés, suivant un ordre prédéterminé. De préférence, l'étape b) est réalisée par l'orthodontiste, de préférence en présence du patient.

Les traitements orthodontiques élémentaires sont de préférence choisis parmi les traitements orthodontiques élémentaires connus de l'art antérieur pour modifier la morphologie de la tête et/ou la configuration des dents, en particulier pour un résultat thérapeutique, prophylactique ou esthétique.

Un bloc de paramétrage est une clé qui, appliquée à un traitement orthodontique élémentaire défini par une fonction générale, par exemple « modifier la position de la mâchoire inférieure », le transforme en une solution opérationnelle, suffisamment définie pour qu'un appareil orthodontique puisse être conçu pour mettre en oeuvre cette solution.

Selon l'invention, le premier traitement orthodontique élémentaire à être paramétré est un traitement orthodontique élémentaire capital, de préférence choisi parmi un traitement de modification de la hauteur de la mâchoire inférieure, un traitement de modification de la largeur de la mâchoire supérieure, et/ou un traitement de modification de la position de la mâchoire inférieure par rapport à la mâchoire supérieure

Le bloc de paramétrage activé doit être compatible avec le règlement. Par exemple, si le règlement impose, pour l'instant final, une position de la mâchoire inférieure, suivant la direction avant-arrière, comprise entre 0 et 5 mm de sa position initiale vers l'avant, le traitement orthodontique complexe comportant le traitement orthodontique élémentaire paramétré avec ce bloc de paramétrage ne devra pas conduire à un déplacement de la mâchoire inférieure qui la conduirait à plus de 5 mm de sa position initiale vers l'avant.

Classiquement, il existe plusieurs blocs de paramétrage compatibles avec le règlement. Par exemple, si le premier traitement orthodontique élémentaire est un traitement orthodontique pour modifier la position de la mâchoire inférieure, suivant la direction avant-arrière, il peut exister plusieurs solutions techniques pour réaliser cette modification, chacune de ces solutions étant le résultat du paramétrage du traitement orthodontique élémentaire avec un bloc de paramétrage correspondant. Pour traiter une malocclusion, il est possible de choisir un traitement avec arc et attaches ou un traitement avec des gouttières orthodontiques, ces deux traitements étant associés à des blocs de paramétrage respectifs, à appliquer au traitement orthodontique élémentaire de « traitement d'une malocclusion ».

Les blocs de paramétrage possibles pour un traitement orthodontique élémentaire, c'est-à-dire les différents traitements orthodontiques élémentaires envisageables, ainsi que les paramètres correspondants, sont bien connus de l'homme de l'art.

De préférence, l'ordinateur détermine un groupe constitué des blocs de paramétrage compatibles avec le règlement, par exemple en consultant une base de données établie à cet effet. De préférence, on présente alors à l'utilisateur, de préférence sur l'écran de l'ordinateur, ces blocs de paramétrage « potentiels ». De préférence, le groupe de blocs de paramétrage potentiels associé au traitement orthodontique élémentaire en cours de traitement est présenté sous forme d'une ou plusieurs listes, à choix multiple ou unique, affichée(s) sur l'écran. L'utilisateur choisit alors un de ces blocs de paramétrage potentiels, c'est-à-dire l'active.

Dans un mode de réalisation préféré, on présente, de préférence en temps réel, de préférence sur l'écran de l'ordinateur, des informations sur l'effet de l'activation d'un bloc de paramétrage potentiel. Par exemple, on affiche sur l'écran d'ordinateur les marges résiduelles de variation de valeurs pour un ou plusieurs paramètres du traitement orthodontique complexe, en particulier pour les paramètres transversaux, de préférence pour la durée et/ou le coût et/ou un coefficient de douleur et/ou un nombre de rendez-vous chez l'orthodontiste relatifs au traitement orthodontique complexe actif.

De préférence encore, on affiche sur l'écran d'ordinateur, de préférence en temps réel, l'effet de l'activation du bloc de paramétrage sur une ou plusieurs valeurs « finales » de paramètres capitaux et/ou dentaires, la valeur finale d'un paramètre étant la valeur auquel le traitement orthodontique complexe, tel que paramétré à l'instant considéré, conduirait à l'instant final. Par exemple, on affiche sur l'écran d'ordinateur la position de la mâchoire inférieure prévue à la fin du traitement orthodontique complexe. Dans un mode de réalisation préféré, cet affichage est graphique. De préférence, on présente une vue d'un modèle 3D de la tête et/ou des dents, de préférence hyperréaliste, illustrant la morphologie finale de la tête et/ou la configuration finale des dents du patient.

De préférence encore, l'ordinateur autorise un retour en arrière dans le déroulement du procédé. En particulier, l'utilisateur peut revenir à une étape antérieure du paramétrage pour supprimer l'activation d'un bloc de paramétrage. L'utilisateur, de préférence l'orthodontiste, peut ainsi tester l'effet de différents blocs de paramétrage potentiels avant de choisir celui qu'il active définitivement.

Ce contrôle en temps réel permet d'accélérer considérablement le paramétrage du traitement orthodontique complexe. Il contribue également à la formation de l'utilisateur, ce dernier apprenant ainsi à évaluer les effets de l'activation des différents blocs de paramétrage. Les étapes a) et b) d'un procédé selon l'invention peuvent être ainsi utilisées pour former un étudiant en orthodontie ou un orthodontiste.

Les traitements orthodontiques élémentaires suivants peuvent être capitaux ou dentaires. Ils sont paramétrés suivant la même procédure que le premier traitement orthodontique élémentaire.

Seuls les blocs de paramétrage compatibles avec le règlement peuvent être sélectionnés. Notamment, un bloc de paramétrage ne peut être activé que s'il n'est pas incompatible avec les traitements orthodontiques élémentaires tels que paramétrés précédemment. A mesure que des traitements orthodontiques élémentaires sont ainsi paramétrés, le nombre de blocs de paramétrage potentiels associés aux traitements orthodontiques élémentaires se réduit donc. Le traitement orthodontique complexe doit en effet toujours rester conforme au règlement.

Par exemple, si le coût maximal pour réaliser le traitement orthodontique complexe, imposé par le règlement, est de 10 000 €, et que l'utilisateur choisit, pour paramétrer le premier traitement orthodontique élémentaire, d'activer un bloc de paramétrage qui engendre un coût de 2 000 €, les blocs de paramétrage potentiels qui seront proposés lors du paramétrage du deuxième traitement orthodontique élémentaire, encore à paramétrer, ne pourront engendrer un coût dépassant 8 000 €.

Autre exemple, si le deuxième traitement orthodontique élémentaire est un traitement orthodontique d'élargissement de la mâchoire inférieure, et que le premier traitement orthodontique élémentaire a conduit à imposer une position de la mâchoire inférieure, suivant la direction avant-arrière, comprise entre 0 et 5 mm de sa position initiale vers l'avant, aucun des blocs de paramétrage potentiels pour le deuxième traitement orthodontique élémentaire ne devra conduire, s'il est activé, à un traitement orthodontique complexe paramétré déplaçant la mâchoire inférieure à plus de 5 mm de sa position initiale vers l'avant, ni à un traitement orthodontique complexe paramétré déplaçant la mâchoire inférieure vers l'arrière.

Les règles capitales et dentaires limitent les variations de valeurs de paramètres capitaux et dentaires, respectivement, pendant le traitement orthodontique complexe, à partir de leurs valeurs initiales. La vérification de la conformité du traitement orthodontique complexe au règlement nécessite donc de comparer des valeurs finales de paramètres au référentiel. Le paramétrage du traitement orthodontique complexe permet de le simuler et de déterminer ces valeurs finales à partir des valeurs initiales de ces paramètres, c'est-à-dire à l'instant initial.

De préférence, au moins une partie des valeurs initiales est déterminée avec les images, de préférence sensiblement en même temps que l'on définit le référentiel, à l'étape a).

Les valeurs initiales pour les paramètres capitaux, sont de préférence déterminées à partir desdites images capitales, c'est-à-dire en utilisant l'information fournie par les images capitales, par exemple par mesure d'une dimension sur les images capitales.

La figure 2 représente un exemple d'image utilisée pour déterminer une valeur initiale pour des paramètres capitaux relatifs à la longueur de la tête (distances *l1, l2, l3*) et relatif au rapport de la hauteur de la mâchoire supérieure sur la hauteur de la mâchoire inférieure (distances *l31 et l32*).

Pour déterminer une valeur initiale à partir d'une image, l'image est de préférence affichée sur l'écran de l'ordinateur, puis l'utilisateur, de préférence un orthodontiste, place une ou plusieurs marques de mesure 10 sur l'image. De préférence, l'ordinateur calcule alors une valeur initiale en fonction de la position des marques de mesure, par exemple la distance entre deux points placés par l'utilisateur. Tous les logiciels connus pour effectuer des mesures sur des images peuvent utilisés.

La marque de mesure peut être notamment une marque géométrique, de préférence choisie parmi un point, une droite ou un cercle.

Par exemple, sur la figure 2, les marques de mesure 10 sont un point inter-sourcilier, un point sous-nasal, un point mentonnier, et un point de l'extrémité supérieure du front. L'ordinateur peut alors déterminer à partir de ces points les hauteurs *l1*, *l2* et *l3.* En ajoutant un point inter-labial, l'ordinateur peut également déterminer les hauteurs *l31* et *l32.*

La figure 3 illustre un exemple de détermination d'une valeur initiale pour une pluralité de paramètres relatifs à la configuration des dents du patient représentées sur trois photos. Des marques de mesure, à savoir des points 101 et des droites 100 ont été placées, automatiquement ou manuellement, sur les images, et des mesures de distance entre ces marques de mesure ont été effectuées par l'ordinateur pour constituer lesdites valeurs initiales.

De préférence, au moins une marque de mesure est choisie dans le groupe constitué par
- un point inter-sourcilier, c'est-à-dire à mi-distance des sourcils, sur une vue de face ;
- un point sous-nasal, c'est-à-dire à mi-distance des narines, sous les narines, sur une vue de face ;
- un point mentonnier, c'est-à-dire le point le plus proéminent à l'extrémité du menton ;
- un point à l'extrémité supérieure du front, c'est-à-dire situé sur la ligne d'implantation des cheveux, au milieu du front,
- un point sur la pointe cuspidienne d'une dent ;
- un point situé au niveau de la jonction de deux dents, de préférence au niveau de la gencive ;
- une droite reliant un point le plus proéminent du front et un point sous-nasal.

Une marque de mesure, par exemple une droite ou un cercle, peut être tracée par l'ordinateur à partir d'autres marques de mesure, par exemple de points saisis par l'utilisateur.

De préférence, des instructions sont données oralement ou affichées sur un écran afin que l'utilisateur positionne les marques de mesure manuellement, par exemple à l'aide d'une souris, d'un stylet ou d'un doigt, en particulier par interaction avec un écran tactile de l'ordinateur.

Les marques de mesure peuvent être placées automatiquement grâce à une méthode d'analyse d'images, par exemple grâce à des méthodes d'apprentissage automatique, en particulier un réseau de neurones. De préférence, les marques de mesure peuvent être déplacées par l'utilisateur, par exemple avec une souris d'ordinateur ou un stylet ou un doigt sur une tablette. De préférence, les valeurs initiales déterminées à partir de ces marques de mesure sont modifiées en conséquence, de préférence en temps réel.

De préférence, les valeurs initiales sont représentées sur l'écran d'ordinateur, et de préférence mises à jour en temps réel lorsque l'utilisateur déplace des marques de mesure.

Bien entendu, les marques de mesure peuvent servir pour préciser, de manière visuelle et très rapidement, plusieurs valeurs initiales.

L'ordre de paramétrage des traitements orthodontiques élémentaires détermine un cadre pour la sélection des blocs de paramétrage qui est variable en fonction du traitement orthodontique élémentaire considéré.

Selon l'invention, au moins un traitement orthodontique élémentaire capital, c'est-à-dire relatif à la morphologie de la tête du patient, est paramétré avant qu'un traitement orthodontique élémentaire dentaire, c'est-à-dire relatif à la configuration des dents, n'ait été paramétré. Ainsi, seuls les blocs de paramétrage qui ne sont pas incompatibles avec ce traitement orthodontique élémentaire capital peuvent être activés lors du paramétrage des traitements orthodontiques élémentaires suivants.

Dans un mode de réalisation, plusieurs, voire tous les traitements orthodontiques élémentaires capitaux sont paramétrés avant qu'un traitement orthodontique dentaire ne soit paramétré.

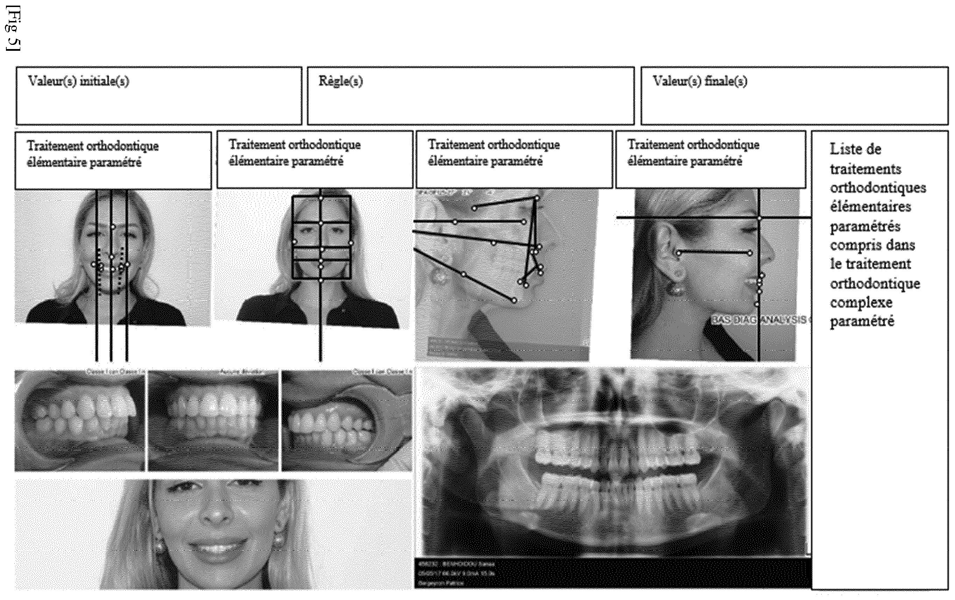

De préférence, à l'issue de l'étape b), on génère un compte rendu comportant des informations relatives au règlement, et/ou aux valeurs initiales et/ou aux blocs de paramétrage activés, et/ou à un appareil orthodontique permettant la mise en oeuvre du traitement orthodontique complexe, et/ou à la configuration des dents estimée pour l'instant final et/ou à la morphologie de la tête du patient estimée pour l'instant final. Ce compte-rendu peut être présenté à l'orthodontiste et/ou au patient, par exemple en étant envoyé ou en étant affiché, par exemple sur l'écran de l'ordinateur. Un exemple de compte-rendu est représenté figure 5.

Le règlement et le paramétrage des traitements orthodontiques élémentaires peuvent ne pas avoir conduit à fixer une valeur pour chaque paramètre. Autrement dit, l'orthodontiste peut encore disposer, pour certains paramètres, de plusieurs options qui conduiront chacune à un traitement orthodontique complexe conforme au règlement. Dans ce cas, l'orthodontiste finalise le paramétrage du traitement orthodontique complexe en déterminant une valeur pour chacun des paramètres encore non fixés.

**A l'étape c**), on conçoit et on fabrique au moins un appareil orthodontique adapté au traitement orthodontique complexe. Ces opérations sont conventionnelles et ne posent aucune difficulté à un orthodontiste.

De préférence, l'appareil orthodontique est une gouttière orthodontique. L'appareil orthodontique peut être également une bague, un fil orthodontique ou tout autre appareil orthodontique connu.

Elle est remise ensuite au patient, qui la dispose dans sa bouche pour suivre le traitement orthodontique complexe.

### Exemple

A un instant initial, de préférence avant un traitement orthodontique, on acquiert au moins une photo capitale du patient vue de face, une photo capitale du patient vue de profil, une photo de sourire vue de face, une photo de sourire vue de profil, des photos des dents du patient vues de gauche, de droite et de face, et une radiologie de la tête du patient vue de face. On acquiert également des photos dentaires, par exemple une vue de face, une vue de profil gauche et une vue de profil droit, un écarteur dentaire étant porté par le patient lors de l'acquisition de ces images dentaires. Ces images peuvent également être acquises à différents instants dans le temps, de préférence les différents instants étant distants de moins de 6 mois, mieux moins de deux mois, encore mieux moins d'un mois. De préférence, on acquiert au moins 5 images dentaires et/ou capitales ; mieux au moins 10 images dentaires et/ou capitales ; encore mieux au moins 15 images dentaires et/ou capitales.

Les images du patient acquises sont ensuite chargées sur un ordinateur.

A l'étape a), l'utilisateur, de préférence l'orthodontiste, de préférence en présence du patient, détermine ensuite les règles du règlement. Le règlement est déterminé, au moins partiellement, à partir desdites images.

L'utilisateur détermine au moins une règle capitale. La figure 2 illustre un exemple de détermination d'une règle capitale à partir des points de référence sur une photo capitale du patient vue de face. L'utilisateur peut alors par exemple déterminer une hauteur maximale *l3ₘₐₓ* et une hauteur minimale *l3ₘᵢₙ* en positionnant des points avec une souris, un stylet ou un doigt sur l'écran de l'ordinateur ou en entrant des valeurs avec un clavier. L'utilisateur saisit par ailleurs des points de mesures permettant à l'ordinateur de déterminer la hauteur de la mâchoire sur les images. Sur la figure 2, la hauteur *l3*, déterminée grâce aux points de mesures 10, en particulier un point mentonnier et un point sous-nasal, représente la hauteur de mâchoire initiale. Une règle capitale peut être définie par « la hauteur finale de mâchoire du patient doit être comprise dans l'intervalle [*l3ₘᵢₙ* ; *l3ₘₐₓ*] ». Une règle peut également être définie en saisissant une variation maximale et une variation minimale par rapport au référentiel. Ainsi, un autre exemple de règle, définissant une contrainte pour la hauteur de la mâchoire du patient peut être « la hauteur finale de mâchoire du patient *l3_{finale}* est comprise entre *l3*+Δₘᵢₙ et *l3*+Δₘₐₓ », Δₘᵢₙ et Δₘₐₓ correspondant à des valeurs maximales acceptables de déplacement de la mâchoire du patient lors du traitement orthodontique complexe.

L'utilisateur détermine ensuite au moins une règle dentaire._La figure 3 illustre un exemple de détermination d'une règle dentaire à partir de trois images dentaires du patient. Une règle dentaire peut en particulier être déterminée grâce aux marques de mesures 100a ; 100b ; 101a et 101b. Une règle peut par exemple être « une droite passant par deux points distincts situés entre les incisives centrales supérieures et une droite passant par deux points distincts situés entre les incisives centrales inférieures doivent être sensiblement confondues à l'issue du traitement orthodontique complexe » ou « une droite passant par deux points distincts situés entre les incisives centrales supérieures et une droite passant par deux points distincts situés entre les incisives centrales inférieures doivent être sensiblement parallèles et la distance séparant ces deux droites doit être inférieure à 1 mm à l'issue du traitement orthodontique complexe ». Par exemple, l'ordinateur détermine automatiquement les droites de mesures 100a et 100b à partir de points de mesures 101a et 101b positionnés par l'utilisateur.

L'utilisateur détermine ensuite au moins une règle transversale. De préférence au moins une règle transversale est déterminée par le patient, et saisie dans l'ordinateur.

Une règle transversale peut en particulier être la détermination d'un coût maximal du traitement orthodontique complexe, par exemple « le coût maximal du traitement orthodontique complexe doit être inférieur à 10 000€ », et d'une durée maximale du traitement orthodontique complexe, par exemple « la durée maximale du traitement orthodontique complexe doit être inférieure ou égale à 1 mois ».

De préférence, l'utilisateur détermine également un coefficient de douleur et/ou de confort pour le traitement orthodontique complexe. Le coefficient de douleur et/ou de confort est notamment relatif au type d'appareil orthodontique. L'utilisateur sélectionne par exemple un seuil de douleur maximal acceptable pour le patient par rapport à une échelle de douleur préétablie, le seuil étant par exemple un chiffre compris entre 0 et 5.

L'utilisateur peut également définir des règles imposant des caractéristiques techniques des appareils orthodontiques à utiliser pour le traitement orthodontique complexe, par exemple « seules des gouttières doivent être utilisées pour le traitement orthodontique complexe ». L'ordre de détermination des règles du règlement peut être modifié. En particulier, l'utilisateur peut commencer par déterminer tout ou partie des règles transversales, puis tout ou partie des règles capitales, puis tout ou partie des règles dentaires.

A l'étape b), après avoir déterminé au moins une partie des règles du règlement, l'utilisateur prépare le traitement orthodontique complexe. Pour ce fait, l'utilisateur sélectionne au moins un traitement orthodontique élémentaire, mieux plusieurs traitements orthodontiques élémentaires, dans une base de modèles de traitements orthodontiques élémentaires puis les ordonne. De préférence, une liste de traitements orthodontiques complexes potentiels constitués chacun d'une série prédéterminée de traitements orthodontiques élémentaires est disponible et l'utilisateur choisit un traitement orthodontique complexe dans cette liste.

Le traitement orthodontique complexe comporte de préférence les traitements orthodontiques élémentaires suivants, classés suivant l'ordre de paramétrage préféré suivant :
- Paramétrage d'un traitement de déplacement haut-bas de la mâchoire inférieure,
- Paramétrage d'un traitement d'élargissement ou de rétrécissement de l'arcade supérieure et/ou de l'arcade inférieure,
- Paramétrage d'un traitement de déplacement avant-arrière de la mâchoire inférieure et/ou de la mâchoire supérieure ;
- Paramétrage d'un traitement de déplacement gauche/droite de la mâchoire inférieure ;
- Paramétrage d'un traitement d'inclinaison de la mâchoire supérieure et/ou de la mâchoire inférieure ;
- Paramétrage d'un traitement de déplacement des dents, de préférence un traitement d'occlusion dentaire, en particulier un traitement de supraclusion, malocclusion ou infraclusion,
- Paramétrage d'un traitement de déplacement des gencives et/ou de déplacement de la lèvre supérieure et/ou de déplacement des dents.

D'autres paramétrages peuvent être ajoutés, de préférence à la fin des paramétrages tels qu'ordonnés ci-dessus, par exemple, un paramétrage de modification de la couleur des dents.

Lors du paramétrage du premier traitement orthodontique élémentaire, des blocs de paramétrage potentiels correspondant respectant le règlement sont présentés à l'utilisateur, de préférence en étant affichés sur un écran d'ordinateur. L'utilisateur sélectionne alors un bloc de paramétrage potentiel, activant ainsi le bloc de paramétrage sélectionné, et/ou définit manuellement une plage de valeurs acceptables.

Par exemple, lors du paramétrage d'un traitement de déplacement haut-bas de la mâchoire inférieure, les blocs de paramétrage potentiels suivant sont affichés : ingression des molaires du bas d'une variation comprise dans un intervalle [dₘᵢₙ ; dₘₐₓ], ingression des molaires du bas d'une variation comprise dans un intervalle [dₘᵢₙ ; dₘₐₓ], égression des molaires du haut d'une variation comprise dans un intervalle [dₘᵢₙ ; dₘₐₓ], égression des molaires du bas d'une variation comprise dans un intervalle [dₘᵢₙ ; dₘₐₓ]. L'utilisateur sélectionne un de ces blocs de paramétrage et renseigne des valeurs acceptables pour dₘᵢₙ et dₘₐₓ.

Alternativement, dₘᵢₙ et dₘₐₓ sont automatiquement définis dans les blocs de paramétrage potentiels, « ingression des molaires du bas d'une variation comprise dans un intervalle [0 mm ; 5 mm] » ou « ingression des molaires du bas d'une variation comprise dans un intervalle [1 mm ; 3 mm] », ces valeurs étant compatibles avec le règlement. Une information relative au résultat fourni par le traitement orthodontique complexe est de préférence affiché sur l'écran, et mis à jour en temps réel, en particulier après l'activation d'un bloc de paramétrage et/ou la sélection d'une plage de valeurs acceptables. L'information peut être relative à l'effet du traitement orthodontique complexe sur la morphologie de la tête et/ou sur la configuration des dents, ou relative au coût ou à la douleur associée au traitement orthodontique complexe.

L'utilisateur peut sélectionner un bloc de paramétrage potentiel, l'activer et ainsi visualiser le résultat de ce choix. L'utilisateur peut ensuite effectuer le paramétrage du traitement orthodontique élémentaire suivant ou au contraire modifier le paramétrage effectué si le résultat ne convient pas.

On procède de même pour le ou les paramétrages des traitements orthodontiques élémentaires suivants, les blocs de paramétrage potentiels étant dépendants du règlement, mais aussi des paramétrages des traitements orthodontiques élémentaires effectués précédemment. Ainsi, plus le paramétrage d'un traitement orthodontique élémentaire est tardif (plus le traitement orthodontique élémentaire est éloigné du premier traitement orthodontique élémentaire), plus nombreuses sont les contraintes que doivent respecter les blocs de paramétrages potentiels.

Après le paramétrage de l'ensemble des traitements orthodontiques élémentaires par l'utilisateur, un compte-rendu comportant de préférence une partie des images du patient, au moins une partie des traitements orthodontiques élémentaires paramétrés, mieux l'ensemble des traitements orthodontiques élémentaires paramétrés, au moins une représentation de la morphologie cible de la tête et/ou de la configuration cible des dents du patient, et/ou les règles du règlement, est présenté à l'utilisateur. Le compte-rendu peut en particulier permettre de visualiser l'effet du traitement orthodontique complexe paramétré sur la tête et/ou les dents du patient, et fournir des indications opérationnelles, destinées à l'orthodontiste, pour l'exécution du traitement.

Il peut exister plusieurs traitements orthodontiques complexes permettant d'atteindre une morphologie cible de la tête et/ou une configuration cible des dents du patient. L'utilisateur, de préférence l'orthodontiste, finalise alors, de préférence à partir du compte-rendu, le paramétrage du traitement orthodontique complexe. Par exemple, le traitement orthodontique complexe peut laisser deux options pour le paramètre « Nature de l'appareil orthodontique » pour le choix de l'appareil orthodontique : « arc et attaches » - « gouttière ». La finalisation du paramétrage conduit à fixer une valeur, par exemple « gouttière ».

A l'issue de l'étape b), on parvient ainsi à la définition d'un traitement orthodontique opérationnel, adapté aux besoins du patient.

A l'étape c), un appareil orthodontique adapté à ce traitement est conçu, automatiquement, par exemple au moyen d'un ordinateur, puis fabriqué.

Alternativement, le compte-rendu est présenté uniquement après l'étape de conception de l'appareil orthodontique et comporte une description d'au moins une partie des traitements orthodontiques élémentaires paramétrés, et/ou une description de l'appareil orthodontique à fabriquer.

Comme cela apparaît clairement à présent, l'invention permet de concevoir un appareil orthodontique adapté pour modifier la configuration des dents, conformément aux appareils orthodontiques conventionnels, mais aussi de garantir que cette modification ne s'accompagnera pas d'une modification inacceptable de la morphologie de la tête, voire, qu'elle s'accompagnera d'une modification de la morphologie de la tête souhaitée par le patient et/ou l'orthodontiste.

Bien entendu, l'invention n'est pas limitée aux modes de réalisation décrits ci-dessus et représentés.

En particulier, au moins une partie du référentiel, en particulier les limites pour les variations des valeurs des paramètres capitaux et dentaires pendant le traitement orthodontique complexe, peuvent être définies, au moins partiellement, avec des modèles 3D, par exemple acquis au moyen d'un scanner. Ces modèles 3D contiennent de préférence un modèle dentaire représentant au moins une partie des dents du patient et/ou un modèle capital, représentant plus de 50%, de préférence plus de 70%, plus de 80%, plus de 90%, de préférence 100% de la tête du patient.

En particulier, le patient n'est pas limité à un être humain. Un procédé selon l'invention peut être utilisé pour un autre animal.

## Revendications

1. Procédé de fabrication d'au moins un appareil orthodontique destiné à être porté par un patient, pour la mise en oeuvre, depuis un instant initial jusqu'à un instant final, d'un traitement orthodontique complexe constitué d'au moins un premier traitement orthodontique élémentaire configuré pour modifier la morphologie de la tête du patient et d'au moins un deuxième traitement orthodontique élémentaire configuré pour modifier la configuration des dents du patient, ledit procédé comportant les étapes suivantes :
a) détermination, de préférence au moyen d'un ordinateur, d'un règlement définissant des règles pour le traitement orthodontique complexe ; puis
b) paramétrage, au moyen d'un ordinateur, dudit au moins un premier traitement orthodontique élémentaire, puis dudit au moins un deuxième traitement orthodontique élémentaire, de manière qu'après ledit paramétrage, le traitement orthodontique complexe respecte toujours lesdites règles ;
c) conception, au moyen d'un ordinateur, et fabrication d'au moins un appareil orthodontique adapté audit traitement orthodontique complexe,
le règlement définissant au moins une règle capitale relative à la morphologie de la tête, au moins une règle dentaire relative à la configuration des dents, et au moins une règle transversale qui n'est ni une règle capitale, ni une règle dentaire,
la au moins une règle capitale portant sur une valeur choisie dans le groupe constitué par une hauteur de visage à l'instant final et une position de la lèvre inférieure et/ou de la lèvre supérieure par rapport au menton et au front,
la au moins une règle transversale portant sur une valeur choisie dans le groupe constitué par
- un coût pour le traitement orthodontique complexe,
- une durée pour le traitement orthodontique complexe,
- un coefficient de douleur pour le traitement orthodontique complexe,
- un coefficient de confort pour le traitement orthodontique complexe,
- une ou plusieurs caractéristiques techniques pour ledit au moins un appareil orthodontique.

2. Procédé selon la revendication immédiatement précédente, dans lequel le traitement orthodontique complexe comporte un traitement orthodontique élémentaire choisi dans le groupe constitué par
- un traitement de déplacement de la mâchoire inférieure vers le haut ou le bas ;
- un traitement de modification de la largeur de la mâchoire supérieure ;
- un traitement de modification de la largeur de la mâchoire inférieure ;
- un traitement de déplacement de la mâchoire inférieure vers l'avant ou vers l'arrière;
- un traitement de déplacement de la mâchoire supérieure vers l'avant ou vers l'arrière ;
- un traitement de déplacement latéral de la mâchoire inférieure ;
- un traitement de modification de l'inclinaison transversale des mâchoires ;
- un traitement de déplacement d'une ou plusieurs dents ;
- un traitement de déplacement des gencives ;
- un traitement de modification de la couleur d'une ou plusieurs dents, de préférence de blanchissement des dents.

3. Procédé selon l'une quelconque des revendications précédentes, dans lequel les traitements orthodontiques élémentaires sont ordonnées pour être paramétrés suivant l'ordre de paramétrage suivant :
- paramétrage d'un traitement de déplacement de la mâchoire inférieure, de préférence comportant un paramétrage d'un traitement de déplacement vertical de la mâchoire inférieure ;
- paramétrage d'un traitement de modification de la largeur de la mâchoire supérieure ;
- paramétrage d'un traitement de modification de la largeur de la mâchoire inférieure ;
- paramétrage d'un traitement de déplacement avant/arrière de la mâchoire inférieure ;
- paramétrage d'un traitement de déplacement avant/arrière de la mâchoire supérieure ;
- paramétrage d'un traitement de déplacement droite/gauche de la mâchoire inférieure ;
- paramétrage d'un traitement d'inclinaison des mâchoires ;
- paramétrage d'un traitement de déplacement d'une ou plusieurs dents ;
- paramétrage d'un traitement de déplacement des gencives ;
- paramétrage d'un traitement de modification de la couleur d'une ou plusieurs dents.

4. Procédé selon l'une quelconque des revendications précédentes, dans lequel le règlement définit :
- au moins une règle capitale relative à la morphologie de la tête portant sur une valeur choisie dans le groupe constitué par
- une hauteur de la mâchoire inférieure et/ou une hauteur de la mâchoire supérieure à l'instant final ;
- une largeur de la mâchoire inférieure à l'instant final,
- une position de la mâchoire inférieure par rapport à la mâchoire supérieure à l'instant final,
- une largeur de la mâchoire supérieure à l'instant final ;
- au moins une règle dentaire relative à la configuration des dents portant sur une valeur choisie dans le groupe constitué par
- une distance par rapport à une configuration cible des dents à l'instant final ;
- une inclinaison par rapport à une configuration cible des dents à l'instant final ;
- un déplacement des gencives par rapport à une configuration cible des dents à l'instant final ;
- une couleur cible des dents à l'instant final.

5. Procédé selon l'une quelconque des revendications précédentes, dans lequel pour vérifier, à un instant de l'étape b), si le traitement orthodontique complexe respecte lesdites règles, on mesure des dimensions, sur des images et/ou des modèles 3D affichés sur un écran d'ordinateur, entre des points remarquables desdites images et/ou desdits modèles 3D, lesdites dimensions constituant des valeurs initiales de paramètres du traitement orthodontique complexe, on simule l'effet du traitement orthodontique complexe, tel que paramétré audit instant de l'étape b), sur lesdites dimensions, entre l'instant initial et l'instant final, de manière à déterminer des valeurs finales pour lesdits paramètres, puis on vérifie si lesdites valeurs finales appartiennent à des plages correspondantes définies par les règles du règlement.

6. Procédé selon la revendication immédiatement précédente, dans lequel, pour déterminer lesdites valeurs initiales
- on identifie des points remarquables sur les images et/ou les modèles 3D, et on dispose des marques de mesure en fonction des points remarquables, ledit ordinateur déterminant lesdites valeurs initiales en fonction de la disposition desdites marques de mesure, ou
- les images et/ou les modèles 3D sont analysées informatiquement au moyen d'une méthode d'apprentissage automatique pour déterminer lesdites valeurs initiales.

7. Procédé selon l'une quelconque des deux revendications immédiatement précédentes, dans lequel, pour définir au moins une borne d'une dite plage relative à une position d'un point remarquable du patient, on dispose sur au moins une dite image et/ou un dit modèle 3D au moins une marque de référence, la position de la marque de référence indiquant une position extrême pour ledit point remarquable à l'instant final, et définissant ainsi ladite borne.

8. Procédé selon l'une quelconque des trois revendications immédiatement précédentes, dans lequel lesdites images comprennent au moins
- une image de face et une image de profil de la tête du patient, et/ou
- une image représentant la bouche du patient en position ouverte et au moins une image représentant la bouche du patient en position fermée, et/ou
- une image représentant la bouche du patient en position de sourire, de préférence deux images représentant la bouche du patient en position de sourire comportant une images de face et une profil, et/ou
- un cliché panoramique, et/ou
- une radiographie de la tête du patient ;
et/ou lesdits modèles 3D comprennent au moins un modèle 3D de la face, et/ou un modèle 3D de l'intérieur de la bouche, dit « intraoral », en particulier modèle 3D de dentition et/ou un cliché tomographique ou « cône beam ».

9. Procédé selon l'une quelconque des revendications précédentes, dans lequel le règlement peut être modifié à tout instant lors du paramétrage des traitements orthodontiques élémentaires, dans la mesure où le traitement orthodontique complexe, tel que paramétré jusqu'à cet instant, reste conforme au règlement.

10. Procédé selon l'une quelconque des revendications précédentes, dans lequel pour paramétrer un traitement orthodontique élémentaire, on choisit un bloc de paramétrage dans un groupe de blocs de paramétrage potentiels potentiellement applicables au traitement orthodontique élémentaire pour le paramétrer, l'ordinateur paramétrant ensuite le traitement orthodontique élémentaire avec ledit bloc de paramétrage, ledit groupe de blocs de paramétrage potentiels ne comportant que des blocs de paramétrage qui, appliqués au traitement orthodontique élémentaire, conduisent à un traitement orthodontique complexe respectant le règlement.

11. Procédé selon l'une quelconque des revendications précédentes, dans lequel à un instant lors du paramétrage, on présente sur un écran d'ordinateur et on met à jour en temps réel des informations relatives
- au règlement, et/ou
- à des paramètres du traitement orthodontique complexe, et/ou
- à la morphologie auquel le traitement orthodontique complexe tel que paramétré audit instant conduirait à l'instant final, et/ou
- à la configuration finale des dents auquel le traitement orthodontique complexe tel que paramétré audit instant conduirait à l'instant final.

12. Procédé selon l'une quelconque des revendications précédentes, dans lequel, à l'étape c), on fabrique une gouttière orthodontique.

13. Procédé selon l'une quelconque des revendications précédentes, dans lequel après l'étape c), on génère un compte rendu comportant des informations relatives
- au règlement, et/ou
- à des paramètres du traitement orthodontique complexe, et/ou
- à la morphologie auquel le traitement orthodontique complexe conduira à l'instant final, et/ou
- à la configuration finale des dents auquel le traitement orthodontique complexe conduira à l'instant final.

14. Procédé selon l'une quelconque des revendications précédentes, dans lequel ledit paramétrage est présenté à un utilisateur pour former ledit utilisateur à l'orthodontie.

15. Procédé selon l'une quelconque des revendications précédentes, dans lequel tout premier traitement orthodontique élémentaire est paramétré avant tout deuxième traitement orthodontique élémentaire.

16. Kit comportant :
- un appareil d'acquisition d'images et/ou de modèles 3D,
- un programme informatique comprenant des instructions de code pour l'exécution des étapes a), b), et c) de la revendication 1, lorsque ledit programme est exécuté par un ordinateur.

## Patentansprüche

1. Verfahren zur Herstellung mindestens einer orthodontischen Vorrichtung, die dazu bestimmt ist, von einem Patienten getragen zu werden, um von einem Anfangszeitpunkt bis zu einem Endzeitpunkt eine komplexe orthodontische Behandlung durchzuführen, die aus mindestens einer ersten elementaren orthodontischen Behandlung, die dazu konfiguriert ist, die Morphologie des Kopfes des Patienten zu modifizieren, und mindestens einer zweiten elementaren orthodontischen Behandlung, die dazu konfiguriert ist, die Konfiguration der Zähne des Patienten zu modifizieren, besteht, wobei das Verfahren die folgenden Schritte umfasst:
a) Bestimmen, vorzugsweise mit Hilfe eines Computers, eines Regelwerks, das Regeln für die komplexe orthodontische Behandlung definiert; dann
b) Parametrieren, mit Hilfe eines Computers, der mindestens einen ersten elementaren orthodontischen Behandlung, dann der mindestens einen zweiten elementaren orthodontischen Behandlung, derart, dass die komplexe orthodontische Behandlung nach der Parametrierung immer noch die Regeln einhält;
c) Entwerfen, mit Hilfe eines Computers, und Herstellen mindestens einer orthodontischen Vorrichtung, die an die komplexe orthodontische Behandlung angepasst ist,
wobei das Regelwerk mindestens eine kopfbezogene Regel hinsichtlich der Morphologie des Kopfes, mindestens eine zahnbezogene Regel hinsichtlich der Konfiguration der Zähne und mindestens eine Nebenregel, die weder eine kopfbezogene Regel noch eine zahnbezogene Regel ist, definiert,
wobei sich die mindestens eine kopfbezogene Regel auf einen Wert bezieht, der aus der Gruppe ausgewählt ist, die aus einer Gesichtshöhe zu dem Endzeitpunkt und einer Position der unteren Lippe und/oder der oberen Lippe mit Bezug auf das Kinn und die Stirn besteht,
wobei sich die mindestens eine Nebenregel auf einen Wert bezieht, der aus der Gruppe ausgewählt ist, die aus Folgendem besteht:
- Kosten für die komplexe orthodontische Behandlung,
- einer Dauer für die komplexe orthodontische Behandlung,
- einem Schmerzkoeffizienten für die komplexe orthodontische Behandlung,
- einem Komfortkoeffizienten für die komplexe orthodontische Behandlung,
- einer oder mehreren technischen Eigenschaften für die mindestens eine orthodontische Vorrichtung.

2. Verfahren nach dem unmittelbar vorhergehenden Anspruch, wobei die komplexe orthodontische Behandlung eine elementare orthodontische Behandlung umfasst, die aus der Gruppe ausgewählt ist, die aus Folgendem besteht:
- einer Behandlung zur Bewegung des Unterkiefers nach oben oder nach unten;
- einer Behandlung zur Modifizierung der Breite des Oberkiefers;
- einer Behandlung zur Modifizierung der Breite des Unterkiefers;
- einer Behandlung zur Bewegung des Unterkiefers nach vorne oder nach hinten;
- einer Behandlung zur Bewegung des Oberkiefers nach vorne oder nach hinten;
- einer Behandlung zur seitlichen Bewegung des Unterkiefers;
- einer Behandlung zur Modifizierung der Querneigung der Kiefer;
- einer Behandlung zur Bewegung eines oder mehrerer Zähne;
- einer Behandlung zur Bewegung des Zahnfleisches;
- einer Behandlung zur Modifizierung der Farbe eines oder mehrerer Zähne, vorzugsweise zur Aufhellung der Zähne.

3. Verfahren nach einem beliebigen der vorhergehenden Ansprüche, wobei die elementaren orthodontischen Behandlungen in eine Reihenfolge gebracht werden, um gemäß der folgenden Parametrierungsreihenfolge parametriert zu werden:
- Parametrierung einer Behandlung zur Bewegung des Unterkiefers, die vorzugsweise eine Parametrierung einer Behandlung zur vertikalen Bewegung des Unterkiefers umfasst;
- Parametrierung einer Behandlung zur Modifizierung der Breite des Oberkiefers;
- Parametrierung einer Behandlung zur Modifizierung der Breite des Unterkiefers;
- Parametrierung einer Behandlung zur Bewegung des Unterkiefers nach vorne/hinten;
- Parametrierung einer Behandlung zur Bewegung des Oberkiefers nach vorne/hinten;
- Parametrierung einer Behandlung zur Bewegung des Unterkiefers nach rechts/links;
- Parametrierung einer Behandlung zur Neigung der Kiefer;
- Parametrierung einer Behandlung zur Bewegung eines oder mehrerer Zähne;
- Parametrierung einer Behandlung zur Bewegung des Zahnfleisches;
- Parametrierung einer Behandlung zur Modifizierung der Farbe eines oder mehrere Zähne.

4. Verfahren nach einem beliebigen der vorhergehenden Ansprüche, wobei das Regelwerk Folgendes definiert:
- mindestens eine kopfbezogene Regel hinsichtlich der Morphologie des Kopfes, die sich auf einen Wert bezieht, der aus der Gruppe ausgewählt ist, die aus Folgendem besteht:
- einer Höhe des Unterkiefers und/oder einer Höhe des Oberkiefers zu dem Endzeitpunkt;
- einer Breite des Unterkiefers zu dem Endzeitpunkt,
- einer Position des Unterkiefers mit Bezug auf den Oberkiefer zu dem Endzeitpunkt;
- einer Breite des Oberkiefers zu dem Endzeitpunkt;
- mindestens eine zahnbezogene Regel hinsichtlich der Konfiguration der Zähne, die sich auf einen Wert bezieht, der aus der Gruppe ausgewählt ist, die aus Folgendem besteht:
- einem Abstand mit Bezug auf eine Zielkonfiguration der Zähne zu dem Endzeitpunkt;
- einer Neigung mit Bezug auf eine Zielkonfiguration der Zähne zu dem Endzeitpunkt;
- einer Bewegung des Zahnfleisches mit Bezug auf eine Zielkonfiguration der Zähne zu dem Endzeitpunkt;
- einer Zielfarbe der Zähne zu dem Endzeitpunkt.

5. Verfahren nach einem beliebigen der vorhergehenden Ansprüche, wobei, um zu einem Zeitpunkt des Schritts b) zu überprüfen, ob die komplexe orthodontische Behandlung die Regeln einhält, auf Bildern und/oder 3D-Modellen, die auf einem Computerbildschirm angezeigt werden, Maße zwischen markanten Punkten der Bilder und/oder der 3D-Modelle gemessen werden, wobei die Maße Anfangswerte von Parametern der komplexen orthodontischen Behandlung darstellen, der Effekt der komplexen orthodontischen Behandlung, wie zu dem Zeitpunkt des Schritts b) parametriert, auf die Maße zwischen dem Anfangszeitpunkt und dem Endzeitpunkt simuliert wird, um Endwerte für die Parameter zu bestimmen, und dann überprüft wird, ob die Endwerte zu entsprechenden Bereichen gehören, die durch die Regeln des Regelwerks definiert werden.

6. Verfahren nach dem unmittelbar vorhergehenden Anspruch, wobei, um die Anfangswerte zu bestimmen:
- markante Punkte auf den Bildern und/oder den 3D-Modellen identifiziert werden und in Abhängigkeit von den markanten Punkten Messmarkierungen angebracht werden, wobei der Computer die Anfangswerte in Abhängigkeit von der Anbringung der Messmarkierungen bestimmt, oder
- die Bilder und/oder die 3D-Modelle mit Hilfe einer Methode zum maschinellen Lernen digital analysiert werden, um die Anfangswerte zu bestimmen.

7. Verfahren nach einem beliebigen der zwei unmittelbar vorhergehenden Ansprüche, wobei, um mindestens eine Grenze eines Bereichs für eine Position eines markanten Punkts des Patienten zu definieren, auf mindestens einem Bild und/oder 3D-Modell mindestens eine Referenzmarkierung angebracht wird, wobei die Position der Referenzmarkierung eine Extremposition für den markanten Punkt zu dem Endzeitpunkt angibt und somit die Grenze definiert.

8. Verfahren nach einem beliebigen der drei unmittelbar vorhergehenden Ansprüche, wobei die Bilder mindestens Folgendes beinhalten:
- ein Bild von vorne und ein Profilbild des Kopfes des Patienten und/oder
- ein Bild, das den Mund des Patienten in geöffneter Position darstellt, und mindestens ein Bild, das den Mund des Patienten in geschlossener Position darstellt, und/oder
- ein Bild, das den Mund des Patienten in lächelnder Position darstellt, vorzugsweise zwei Bilder, die den Mund des Patienten in lächelnder Position darstellen und ein Bild von vorne und ein Profilbild umfassen, und/oder
- eine Panoramaaufnahme und/oder
- eine Röntgenaufnahme des Kopfes des Patienten; und/oder wobei die 3D-Modelle mindestens ein 3D-Modell der Vorderseite und/oder ein 3D-Modell des Inneren des Mundes, als "intraoral" bezeichnet, insbesondere ein 3D-Gebissmodell und/oder eine Tomographieaufnahme oder "Cone Beam", beinhalten.

9. Verfahren nach einem beliebigen der vorhergehenden Ansprüche, wobei das Regelwerk zu jedem Zeitpunkt während der Parametrierung der elementaren orthodontischen Behandlungen modifiziert werden kann, vorausgesetzt, dass die komplexe orthodontische Behandlung, wie sie bis zu diesem Zeitpunkt parametriert wurde, zu dem Regelwerk konform bleibt.

10. Verfahren nach einem beliebigen der vorhergehenden Ansprüche, wobei, um eine elementare orthodontische Behandlung zu parametrieren, ein Parametrierungsblock aus einer Gruppe potentieller Parametrierungsblöcke, die auf die elementare orthodontische Behandlung zwecks ihrer Parametrierung potentiell angewendet werden können, ausgewählt wird, wobei der Computer die elementare orthodontische Behandlung im Anschluss mit dem Parametrierungsblock parametriert, wobei die Gruppe potentieller Parametrierungsblöcke nur Parametrierungsblöcke umfasst, die, wenn sie auf die elementare orthodontische Behandlung angewendet werden, zu einer komplexen orthodontischen Behandlung führen, die das Regelwerk einhält.

11. Verfahren nach einem beliebigen der vorhergehenden Ansprüche, wobei zu einem Zeitpunkt während der Parametrierung Informationen über Folgendes auf einem Computerbildschirm präsentiert und in Echtzeit aktualisiert werden:
- das Regelwerk und/oder
- Parameter der komplexen orthodontischen Behandlung und/oder
- die Morphologie, zu der die komplexe orthodontische Behandlung, wie sie zu diesem Zeitpunkt parametriert ist, zu dem Endzeitpunkt führen würde, und/oder
- die Endkonfiguration der Zähne, zu der die komplexe orthodontische Behandlung, wie sie zu diesem Zeitpunkt parametriert ist, zu dem Endzeitpunkt führen würde.

12. Verfahren nach einem beliebigen der vorhergehenden Ansprüche, wobei in Schritt c) ein orthodontischer Aligner hergestellt wird.

13. Verfahren nach einem beliebigen der vorhergehenden Ansprüche, wobei nach Schritt c) ein Protokoll erstellt wird, das Informationen über Folgendes umfasst:
- das Regelwerk und/oder
- Parameter der komplexen orthodontischen Behandlung und/oder
- die Morphologie, zu der die komplexe orthodontische Behandlung zu dem Endzeitpunkt führen wird, und/oder
- die Endkonfiguration der Zähne, zu der die komplexe orthodontische Behandlung zu dem Endzeitpunkt führen wird.

14. Verfahren nach einem beliebigen der vorhergehenden Ansprüche, wobei die Parametrierung einem Benutzer präsentiert wird, um den Benutzer in der Zahnregulierung zu schulen.

15. Verfahren nach einem beliebigen der vorhergehenden Ansprüche, wobei jegliche erste elementare orthodontische Behandlung vor jeglicher zweiten elementaren orthodontischen Behandlung parametriert wird.

16. Kit, das Folgendes umfasst:
- eine Vorrichtung zur Erfassung von Bildern und/oder 3D-Modellen,
- ein EDV-Programm, das Codeanweisungen zur Ausführung der Schritte a), b) und c) von Anspruch 1 beinhaltet, wenn das Programm von einem Computer ausgeführt wird.

## Claims

1. Method for manufacturing at least one orthodontic appliance intended to be worn by a patient, for the implementation, from an initial instant to a final instant, of a complex orthodontic treatment consisting of at least one first basic orthodontic treatment configured to change the morphology of the patient's head and at least one second basic orthodontic treatment configured to change the configuration of the patient's teeth, said method comprising the following steps:
a) determination, preferably by means of a computer, of a regulation defining rules for the complex orthodontic treatment; then
b) parameterization, by means of a computer, of said at least one first basic orthodontic treatment, then of said at least one second basic orthodontic treatment, in such a way that, after said parameterization, the complex orthodontic treatment still complies with said rules;
c) design, by means of a computer, and manufacture of at least one orthodontic appliance adapted to said complex orthodontic treatment,
the regulation defining at least one capital rule relating to the morphology of the head, at least one dental rule relating to the configuration of the teeth, and at least one transverse rule which is neither a capital rule nor a dental rule,
the at least one capital rule concerning a value chosen from the group consisting of a height of the face at the final instant and a position of the lower lip and/or of the upper lip with respect to the chin and the forehead, the at least one transverse rule concerning a value chosen from the group consisting of
- a cost for the complex orthodontic treatment,
- a duration for the complex orthodontic treatment,
- a pain coefficient for the complex orthodontic treatment,
- a comfort coefficient for the complex orthodontic treatment,
- one or more technical characteristics for said at least one orthodontic appliance.

2. Method according to the immediately preceding claim, wherein the complex orthodontic treatment comprises a basic orthodontic treatment chosen from the group consisting of
- a treatment for moving the lower jaw up or down;
- a treatment for changing the width of the upper jaw;
- a treatment for changing the width of the lower jaw;
- a treatment for moving the lower jaw forwards or backwards;
- a treatment for moving the upper jaw forwards or backwards;
- a treatment for moving the lower jaw laterally;
- a treatment for changing the transverse tilt of the jaws;
- a treatment for moving one or more teeth;
- a treatment for moving the gums;
- a treatment for changing the colour of one or more teeth, preferably a teeth whitening treatment.

3. Method according to either of the preceding claims, wherein the basic orthodontic treatments are placed in order so as to be parameterized in the following order of parameterization:
- parameterization of a treatment for moving the lower jaw, preferably comprising a parameterization of a treatment for moving the lower jaw vertically;
- parameterization of a treatment for changing the width of the upper jaw;
- parameterization of a treatment for changing the width of the lower jaw;
- parameterization of a treatment for moving the lower jaw forwards/backwards;
- parameterization of a treatment for moving the upper jaw forwards/backwards;
- parameterization of a treatment for moving the lower jaw to the right/left;
- parameterization of a treatment for tilting the jaws;
- parameterization of a treatment for moving one or more teeth;
- parameterization of a treatment for moving the gums;
- parameterization of a treatment for changing the colour of one or more teeth.

4. Method according to any one of the preceding claims, wherein the regulation defines:
- at least one capital rule relating to the morphology of the head, concerning a value chosen from the group consisting of
- a height of the lower jaw and/or a height of the upper jaw at the final instant;
- a width of the lower jaw at the final instant,
- a position of the lower jaw in relation to the upper jaw, at the final instant,
- a width of the upper jaw at the final instant;
- at least one dental rule relating to the configuration of the teeth, concerning a value chosen from the group consisting of
- a distance with respect to a target configuration of the teeth at the final instant;
- a tilt with respect to a target configuration of the teeth at the final instant;
- a movement of the gums with respect to a target configuration of the teeth at the final instant;
- a target colour of the teeth at the final instant.

5. Method according to any one of the preceding claims, wherein in order to check, at an instant in step b), whether the complex orthodontic treatment complies with said rules, dimensions, in images and/or 3D models displayed on a computer screen, are measured between noteworthy points in said images and/or in said 3D models, said dimensions constituting initial values of parameters of the complex orthodontic treatment, the effect of the complex orthodontic treatment, as parameterized at said instant in step b), is simulated on said dimensions, between the initial instant and the final instant, so as to determine final values for said parameters, then it is checked whether said final values fall within corresponding ranges defined by the rules of the regulation.

6. Method according to the immediately preceding claim, wherein, in order to determine said initial values,
- noteworthy points are identified in the images and/or the 3D models, and measurement marks are arranged as a function of the noteworthy points, said computer determining said initial values as a function of the arrangement of said measurement marks, or
- the images and/or the 3D models are analysed by computer using a machine learning method in order to determine said initial values.

7. Method according to either of the two immediately preceding claims, wherein, in order to define at least one limit of a said range relating to a position of a noteworthy point of the patient, at least one reference mark is arranged in at least one of said images and/or one of said 3D models, the position of the reference mark indicating an extreme position for said noteworthy point at the final instant, and thus defining said limit.

8. Method according to any one of the three immediately preceding claims, wherein said images comprise at least
- an image from the front and a profile image of the patient's head, and/or
- an image showing the patient's mouth in the open position and at least one image showing the patient's mouth in the closed position, and/or
- an image showing the patient's mouth in the smiling position, preferably two images showing the patient's mouth in the smiling position comprising an image from the front and a profile, and/or
- a panoramic image, and/or
- an X-ray of the patient's head;
and/or said 3D models comprise at least a 3D model of the face, and/or a 3D model of the inside of the mouth, referred to as "intraoral", in particular a 3D model of the teeth and/or a tomographic image or "cone beam" scan.

9. Method according to any one of the preceding claims, wherein the regulation may be modified at any instant during the parameterization of the basic orthodontic treatments, as long as the complex orthodontic treatment, as parameterized up to this instant, complies with the regulation.

10. Method according to any one of the preceding claims, wherein in order to parameterize a basic orthodontic treatment, a parameterization block is chosen from a group of potential parameterization blocks potentially applicable to the basic orthodontic treatment so as to parameterize same, the computer then parameterizing the basic orthodontic treatment with said parameterization block, said group of potential parameterization blocks comprising only parameterization blocks which, when applied to the basic orthodontic treatment, result in a complex orthodontic treatment that complies with the regulation.

11. Method according to any one of the preceding claims, wherein, at an instant during the parameterization, information is displayed on a computer screen and is updated in real time, this information relating to
- the regulation, and/or
- parameters of the complex orthodontic treatment, and/or
- the morphology resulting, at the final instant, from the complex orthodontic treatment as parameterized at said instant, and/or
- the final configuration of the teeth resulting, at the final instant, from the complex orthodontic treatment as parameterized at said instant.

12. Method according to any one of the preceding claims, wherein, in step c), an orthodontic aligner is manufactured.

13. Method according to any one of the preceding claims, wherein, after step c), a report is generated comprising information relating to
- the regulation, and/or
- parameters of the complex orthodontic treatment, and/or
- the morphology resulting, at the final instant, from the complex orthodontic treatment, and/or
- the final configuration of the teeth resulting, at the final instant, from the complex orthodontic treatment.

14. Method according to any one of the preceding claims, wherein said parameterization is displayed to a user in order to train said user in orthodontics.

15. Method according to any one of the preceding claims, wherein any first basic orthodontic treatment is parameterized before any second basic orthodontic treatment.

16. Kit comprising:
- an appliance for acquiring images and/or 3D models,
- a computer program comprising code instructions for the execution of steps a), b) and c) of Claim 1, when said program is executed by a computer.
